# EUROPEAN PATENT APPLICATION

(11) **EP 4 530 984 A1**
(43) Date of publication of application: **02.04.2025**
(21) Application number: 23199965.7
(22) Date of filing: 27.09.2023
(51) Int. Cl.: G06T 7/73, A61B 6/00

(54) **PATIENT POSE DETERMINATION SYSTEM, MEDICAL IMAGING SYSTEM AND RELATED METHOD**

(71) Applicant: Koninklijke Philips N.V., 5656 AG Eindhoven (NL)
(72) Inventor: KOEPNICK, Johannes, 5656AG Eindhoven (NL); LUNDT, Bernd, 5656AG Eindhoven (NL); MAY, Jan Marek, 5656AG Eindhoven (NL)
(74) Representative: Philips Intellectual Property & Standards

(57) **Abstract**

The invention relates to a patient pose determination system (3), comprising a depth camera (5), a processing unit (6) and an output unit (7). The depth camera (5) is configured to acquire a depth image (17) of a part of interest of a patient (8) or another part of the patient (8) pertaining to the part of interest, the processing unit (6) is configured to determine a pose of the part of interest using the depth image (17) and the output unit (7) is configured to output the pose of the part of interest. The invention further relates to a medical imaging system (1) comprising said patient pose determination system (3) and a medical imaging device (2), to a method (10) of determining a patient pose, to a computer program product and to a computer-readable medium.

## Description

### FIELD OF THE INVENTION

The present invention relates to medical imaging, in particular to a patient pose determination system for medical imaging, a medical imaging system with said patient pose determination system and a method of determining a patient pose.

### BACKGROUND OF THE INVENTION

In medical imaging, in particular in X-ray imaging, there is often a need to take different views of different anatomical features of patients. The acquired X-ray images are annotated with respect to the view of the body part. Anterior-posterior view (AP view) is obtained with a patient facing the X-ray tube of an X-ray imaging device with the cassette/detector behind the patient. The posterior-anterior view (PA view) is obtained with the patient facing the cassette/detector and with the X-ray tube behind the patient. Different views can be taken for better visualizing the different anatomical features and sometimes can depend on the patient condition. R (right) and L (left) annotations of the X-ray image are used to determine the anatomical side of the body. This helps doctors and radiologists to interpret the X-ray images and provide diagnosis for planning the treatment on the correct side of the patient.

For example, when X-ray imaging a head of the patient, skull PA or skull AP images can be acquired. And when imaging a hand, PA or AP images can be acquired for the left or right hand. However, it can be difficult to tell from the X-ray image itself what the view was or indeed whether it was a right hand AP or a left hand PA view for example. In other words, the pose of the body part cannot easily be determined from the acquired X-ray image itself. Therefore, normally a user needs to manually annotate the image. This is done with a metal symbol (L=left, R=right, AP, PA, or Lat=lateral) such as a lead marker placed within the field of view prior to acquisition of the X-ray image or a manual software annotation of the acquired X-ray image using image processing software. However, this interrupts the user workflow and errors can be made where a marker is placed incorrectly or whether post acquisition annotation is incorrect. There are also certain conditions that could lead to wrong-sided annotations of X-ray images, like: situs inversus totalis; dextro-cardia; and right sided pneumoperitoneum. In these conditions, the heart and sometimes other organs appear to be on the right side, as these are marks for detection of anatomical side in the X-ray images. Therefore, there is the potential that this could lead into wrong sided annotations, thus resulting in the wrong diagnosis, wrong sided intervention or sometimes unnecessary intervention leading to iatrogenic injuries and that could also be fatal in some cases. These also lead to increasing the cost and time of patient care.

A solution to the above problems has been presented in international patent application WO 2021/043655 A1, which discloses the use of an optical camera to acquire optical images of a patient and use these images to determine a pose of a body part of the patient. However, in some cases, these detections are difficult, e.g., when the patient's skin is covered by clothing.

### SUMMARY OF THE INVENTION

It is therefore an object of the present invention to provide an improved system for determining the pose of a patient, a medical imaging system with such a system, and a method of determining a patient pose.

The object of the present invention is solved by the subject matter of the independent claims, wherein further embodiments are incorporated in the dependent claims.

In an aspect of the present invention, a patient pose determination system is provided. The system comprises a depth camera, a processing unit and an output unit.

The depth camera is configured to acquire a depth image of a part of interest of a patient or another part of the patient pertaining to the part of interest. In this context, the depth camera may be based on a time-of-flight measurement, in particular operating with visible light or infrared light. Alternatively, or additionally, the depth camera may be based on stereo triangulation, sheet-of-light triangulation or structured light. The depth image may also be called depth map or range image and may be a two-dimensional image comprising information on distances to points in a scene. In particular, every pixel of the depth image may correspond to a distance to the respective point in the scene, wherein the distance may be encoded as a grayscale value of the pixel. Of course, the depth camera may acquire more than one depth image or even a depth video, further improving the accuracy of the system. In particular, the depth image(s) may be acquired at a time when a medical image is acquired of the patient, or shortly before or thereafter, such that the depth image and the medical image correspond to one another.

The part of interest of a patient may be selected by a user, e.g., from a list of possible parts of interest. Alternatively, or additionally, the part of interest may be selected by placing the patient and/or the depth camera such that the depth image covers the part of interest. Alternatively, or additionally, the system may receive information on the part of interest from a medical imaging device, where some information on the part of interest has been provided for medical imaging purposes. In some cases, the pose of the part of interest may be difficult to determine. As an example, a forearm may be considered as part of interest. The pose of the forearm is difficult to determine by looking at the forearm itself. In this case, the depth image may be taken also of the elbow and/or the wrist, which allow a better determination of the pose of the forearm. The elbow and/or the wrist are then examples for other parts of the patient pertaining to the part of interest. Usually, the parts of the patient pertaining to the part of interest are close or adjacent to the parts of interest.

The processing unit is configured to determine a pose of the part of interest using the depth image. In particular, the processing unit may use regions of the depth image that correspond to the part of interest and/or to the other part of the patient. The processing unit may be a computing unit, comprising one or several processors.

The output unit is configured to output the pose of the part of interest. With said automatically determined pose of the part of interest, an interruption of a workflow of medical staff due to adding manual annotations is avoided. Further, missing annotations or wrong annotations are avoided. Also, lead markers, which are, e.g., critical regarding hospital hygiene, are no longer needed. Further, anatomies that are covered by clothes may be correctly analyzed. In optical images, single-colored clothes provide very little contrast and little to no features that may be used to determine the pose of the part of interest. On the other hand, the depth images contain information on the shape of body parts even when the anatomies are covered by clothes. Hence, the depth images contain valuable information for the determination of the pose of the part of interest. Moreover, compared to optical images, the depth images contain little to no patient specific information, making it easier to fulfil data privacy requirements.

According to an embodiment, the pose of the part of interest comprises an orientation of the part of interest, a laterality of the part of interest and/or a view position of the part of interest. In this context, the orientation may comprise a direction in which the part of interest is oriented, e.g., pointing to the top, left, right, or bottom of the image, pointing in the direction of the image or away from the image. The laterality refers to paired body part and may be, in particular, indicated by "left" or "right", e.g., left hand or right hand. Finally, the view position may be an anterior-posterior view (AP view), a posterior-anterior view (PA view), or a lateral view. The pose of the part of interest comprises enough information to correctly interpret the corresponding medical image.

According to an embodiment, the determination of the pose comprises utilization of an artificial intelligence. In particular, such an artificial intelligence may be very powerful and may be able to determine the pose of a plurality of different parts of interest. More particularly, the artificial intelligence may use features and combinations of features in the depth image to determine the pose of the part of interest that are otherwise not easily quantifiable. Further, once the artificial intelligence has been set up, it can be easily adapted for different parts of interest, e.g., by training the artificial intelligence with training data pertaining to said parts of interest.

According to an embodiment, the artificial intelligence is a trained neural network, in particular a trained convolutional neural network, more particularly a trained fully convolutional neural network. Neural networks are very powerful and can perform the pose determination quickly. In particular, using neural networks, the processing of the depth images, in particular the determination of the pose of the part of interest, may be performed in real-time, such that the determination of the pose of the part of interest does not waste any time of the patient, the clinical staff, and the medical imaging device.

According to an embodiment, the trained neural network has been trained on the basis of depth images of a plurality of parts of interest or other parts in a plurality of known poses. As an example, the depth images may have been labeled with the respective poses. By training the neural network with depth images of a plurality of parts of interest, it becomes good at recognizing the pose of the part of interest in new depth images. That is, the higher the number of depth images used for training the neural network, the better and more reliable the results of the determination of the pose of the part of interest. According to an embodiment, the determination of the pose comprises utilization of a model of the part of interest or the other part. Said model may be, e.g., a three-dimensional description of the part of interest or the other part. Depending on the part of interest, anatomy-specific characteristics may be extracted from the depth image. These characteristics are then compared to the corresponding characteristics in the model of the part of interest or the other part, and based on said comparison, the pose of the part of interest is determined.

According to an embodiment, the determination of the pose comprises detection of landmarks within the depth image. Said landmarks may be anatomy-specific features that can be easily distinguished from one another. Examples for said landmarks are the nose and the ears for the head, the shoulder, elbow, and fingers for the arm and the knee and the ankle for the leg. By identifying said landmarks, a good identification of the pose is possible.

According to an embodiment, the determination of the pose comprises analysis of at least one height profile obtained from the depth image. Said height profile may be taken along a straight line in the depth image, but also along a curved path. The height profile may be analyzed very easily, such that an efficient processing unit may be chosen and/or the result of the pose determination may be obtained very quickly. In particular, the height profile is a one-dimensional function, and an analysis of said one-dimensional function may be performed very quickly, e.g., by determining peaks, troughs, and/or radii of curvature. Hence, even a simple processing unit can perform said analysis of the height profile to determine the pose of the part of interest.

According to an embodiment, outputting the pose of the part of interest comprises displaying information on the pose on a display. This displayed pose information may then be used by medical staff to verify that the part of interest has the correct pose. For example, the pose information may be displayed before a medical scan is performed, such that in that case of an incorrect pose, the medical scan can be delayed until the part of interest has the correct pose, hence preventing wrong examinations and therefore reducing the amount of radiation for the patient as well as reducing costs. Alternatively, or additionally, the pose of the part of interest may be output to a medical imaging device, which then compares the determined pose against an expected pose of the part of interest. If it is determined that there is a mismatch between the determined pose and the expected pose, said mismatch may be indicated and/or a start of a medical scan may be prevented.

Alternatively, or additionally, the pose of the part of interest may be used by patient positioning correction software to correct the pose of the part of interest.

Alternatively, or additionally, the pose of the part of interest may be output to the medical imaging device such that the obtained medical images are annotated with the determined pose.

According to an embodiment, the system further comprises an optical camera. Said optical camera is configured to acquire an optical image of the part of interest or the other part of the patient. The processing unit then determines the pose of the part of interest based on both the depth image and the optical image. Since the optical image comprises additional and/or complementary information to the depth image, the determination of the pose of the part of interest is further improved, in particular, the accuracy of said determination is improved. Further, since many depth cameras are already integrated with optical cameras, e.g., as RGB-D cameras, there is no additional cost for the optical camera.

According to an embodiment, the optical camera is a color camera, e.g., an RGB camera, a black-and-white camera and/or an infrared camera. The images taken by any of these cameras help improving the determination of the pose of the part of interest. In particular, the color camera provides a lot of additional information, whereas the infrared camera may contain less patient-specific information, making it easier to fulfil data privacy requirements.

In another aspect of the present invention, a medical imaging system is provided. Said medical imaging system comprises a patient pose determination system according to the above description, and a medical imaging device configured to acquire a medical image of the part of interest. In particular, the patient pose determination system may be integrated with the medical imaging device to form the medical imaging system.

The processing unit is further configured to annotate the medical image with the pose. Said annotation may be performed by adding the detected pose to image meta data of the medical image. Alternatively, a text box containing the pose information may be inserted in the medical image, or the file name of the medical image may be adjusted to contain the pose information. The output unit is then configured to output the pose annotated medical image. Since the determination of the pose and the annotation are automatically performed, an interruption of a workflow of medical staff due to adding manual annotations is avoided. Further, missing annotations or wrong annotations are avoided. Also, lead markers, which are, e.g., critical regarding hospital hygiene, are no longer needed. Further, anatomies that are covered by clothes may be correctly analyzed. Moreover, compared to optical images, the depth images contain little to no patient specific information, making it easier to fulfil data privacy requirements.

The processing unit may be a combined processing unit for the medical imaging device and the patient pose determination system, such that the medical imaging system contains only one processing unit. However, it is also possible that the patient pose determination system and the medical imaging device have separate processing units. In this case, the patient pose information may be sent from the patient pose determination system to the medical imaging device, such that the medical imaging device may annotate the medical images. Alternatively, the medical images may be sent from the medical imaging device to the patient pose determination system, such that the annotation may be performed by the processing unit of the patient pose determination system.

According to an embodiment, the medical imaging device is an X-ray imaging device, a computed tomography (CT) imaging device, and/or a magnetic resonance imaging (MRI) device. Any of these medical imaging devices may benefit from the proper determination of the pose of the part of interest provided by the patient pose determination system. In particular, the X-ray imaging device may be a mobile or a fixed radiographic system and/or a fluoroscopic system.

According to an embodiment, the imaging axis of the depth camera is known with respect to the imaging axis of the medical imaging device. In this case, the pose of the part of interest may be determined with respect to the imaging axis of the medical imaging device. That is, the relative orientation of the depth camera with respect to the medical imaging device is known, and the pose of the part of interest is determined based on said relative orientation. For example, when determining the view position, e.g., AP view or PA view, it is important to take into account whether the depth camera is oriented parallel, anti-parallel, or at another angle to the imaging axis of the medical imaging device.

Further, any of the above-described embodiments of the patient pose determination system may be used in the medical imaging system, wherein the advantages described above apply similarly to the medical imaging system.

In yet another aspect of the present invention, a method of determining a patient pose is provided. According to the method, a depth image of a part of interest of a patient or another part of the patient pertaining to the part of interest is acquired by a depth camera. Then, a pose of the part of interest is determined by a processing unit, using the depth image. Finally, the pose of the part of interest is output by an outputting unit. Since the determination of the pose and the annotation are automatically performed, an interruption of a workflow of medical staff due to adding manual annotations is avoided. Further, missing annotations or wrong annotations are avoided. Also, lead markers, which are, e.g., critical regarding hospital hygiene, are no longer needed. Further, anatomies that are covered by clothes may be correctly analyzed. Moreover, compared to optical images, the depth images contain little to no patient specific information, making it easier to fulfil data privacy requirements.

Details and further embodiments of this method correspond to those given in the above description of the patient pose determination system.

According to an embodiment, the method further comprises acquiring a medical image of the part of interest by a medical imaging device. Then, the medical image is annotated, by the processing unit, with the determined pose, and the pose annotated medical image is output. Further details and advantages of this embodiment correspond to those described above for the medical imaging system.

In yet another aspect of the present invention, a computer program product is provided. The computer program product comprises instructions which, when the program is executed by a computer, cause the computer to carry out the following steps. First, a depth image of a part of interest of a patient or another part of the patient pertaining to the part of interest is received from a depth camera. Then, using said depth image, a pose of the part of interest is determined. Hence, the computer program product allows an automatic determination of the pose, such that an interruption of a workflow of medical staff due to adding manual annotations is avoided. Further, missing annotations or wrong annotations are avoided. Also, lead markers, which are, e.g., critical regarding hospital hygiene, are no longer needed. Further, anatomies that are covered by clothes may be correctly analyzed. Moreover, compared to optical images, the depth images contain little to no patient specific information, making it easier to fulfil data privacy requirements.

Details and further embodiments of the computer program product correspond to those given in the above description of the patient pose determination system and the method of determining a patient pose.
In yet another aspect of the present invention, a computer-readable medium is provided. The computer-readable medium has stored thereon the computer program product according to the above description. Details and further embodiments of the computer-readable medium correspond to those of the computer program product as well as those given in the above description of the patient pose determination system and the method of determining a patient pose.

It shall be understood that a preferred embodiment of the invention can also be any combination of the dependent claims with the respective independent claim. Further, it has to be noted that embodiments of the invention are described with reference to different subject matters. In particular, some embodiments are described with reference to device type claims whereas other embodiments are described with reference to method type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic view of an embodiment of a medical imaging system;
Fig. 2 shows a schematic view of another embodiment of a medical imaging system;
Fig. 3 shows a flowchart of an embodiment of a method of determining a patient pose;
Fig. 4 shows a flowchart of another embodiment of a method of determining a patient pose;
Figs. 5a and 5b show optical images of a left foot and a right foot, respectively; and
Figs. 6a and 6b show depth images of a left foot and a right foot, respectively, along with corresponding height profiles.

In the Figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

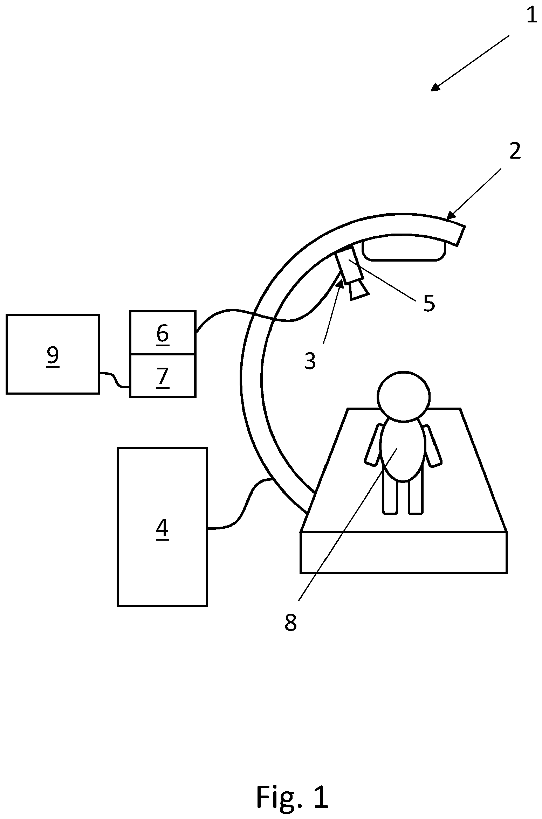

Fig. 1 shows a schematic view of an embodiment of a medical imaging system 1 with a medical imaging device 2 and a patient pose determination system 3.

The medical imaging device 2 is shown as X-ray imaging device, however, other medical imaging devices 2 such as computed tomography (CT) imaging devices and magnetic resonance imaging (MRI) devices may also be used. The medical imaging device 2 has a control unit 4 that controls the operation of the medical imaging device 2.

The patient pose determination system 3 comprises a depth camera 5, a processing unit 6 and an output unit 7. The depth camera 5 is configured to acquire a depth image of a part of interest of a patient 8 and/or of another part of the patient 8 that pertains to the part of interest. In this context, the depth camera 5 may be based on a time-of-flight measurement, in particular operating with visible light or infrared light. Alternatively, or additionally, the depth camera 5 may be based on stereo triangulation, sheet-of-light triangulation or structured light. The depth image may also be called depth map or range image and may be a two-dimensional image comprising information on distances to points in a scene. In particular, every pixel of the depth image may correspond to a distance to the respective point in the scene, wherein the distance may be encoded as a grayscale value of the pixel. Of course, the depth camera 5 may acquire more than one depth image or even a depth video, further improving the accuracy of the system. In particular, the depth image(s) may be acquired at a time when a medical image is acquired of the patient, or shortly before or thereafter, such that the depth image and the medical image correspond to one another. The part of interest of the patient 8 may be selected by a user, e.g., from a list of possible parts of interest. Alternatively, or additionally, the part of interest may be selected by placing the patient 8 and/or the depth camera 5 such that the depth image covers the part of interest. Alternatively, or additionally, the system may receive information on the part of interest from a medical imaging device 2, where some information on the part of interest has been provided for medical imaging purposes. In some cases, the pose of the part of interest may be difficult to determine. As an example, a forearm may be considered as part of interest. The pose of the forearm is difficult to determine by looking at the forearm itself. In this case, the depth image may be taken also of the elbow and/or the wrist, which allow a better determination of the pose of the forearm. The elbow and/or the wrist are then examples for other parts of the patient 8 pertaining to the part of interest. Usually, the parts of the patient 8 pertaining to the part of interest are close or adjacent to the parts of interest.

The depth image is then used by the processing unit 6 to determine a pose of the part of interest, in particular by using regions of the depth image that correspond to the part of interest and/or to the other part of the patient 8. In this context, "pose" may refer to an orientation of the part of interest, a laterality of the part of interest and/or a view position of the part of interest. The orientation may comprise a direction in which the part of interest is oriented, e.g., pointing to the top, left, right, or bottom of the image, pointing in the direction of the image or away from the image. The laterality refers to paired body part and may be, in particular, indicated by "left" or "right", e.g., left hand or right hand. Finally, the view position may be an anterior-posterior view (AP view), a posterior-anterior view (PA view), or a lateral view.

The pose of the part of interest is then output by the output unit 7. With said automatically determined pose of the part of interest, an interruption of a workflow of medical staff due to adding manual annotations is avoided. Further, missing annotations or wrong annotations are avoided. Also, lead markers, which are, e.g., critical regarding hospital hygiene, are no longer needed. Further, anatomies that are covered by clothes may be correctly analyzed. In optical images, single-colored clothes provide very little contrast and little to no features that may be used to determine the pose of the part of interest. On the other hand, the depth images contain information on the shape of body parts even when the anatomies are covered by clothes. Hence, the depth images contain valuable information for the determination of the pose of the part of interest. Moreover, compared to optical images, the depth images contain little to no patient specific information, making it easier to fulfil data privacy requirements.
In the present embodiment, the pose of the part of interest is shown on a screen 9, i.e., on a display, such that medical staff can, e.g., check whether the pose of the part of interest is correct before starting a medical scan.

In an embodiment not shown here, the patient pose determination system 3 may further comprises an optical camera. Said optical camera is configured to acquire an optical image of the part of interest or the other part of the patient 8. The processing unit 6 then determines the pose of the part of interest based on both the depth image and the optical image. Since the optical image comprises additional and/or complementary information to the depth image, the determination of the pose of the part of interest is further improved, in particular, the accuracy of said determination is improved. In this context, the optical camera may be a color camera, e.g., an RGB camera, a black-and-white camera and/or an infrared camera.

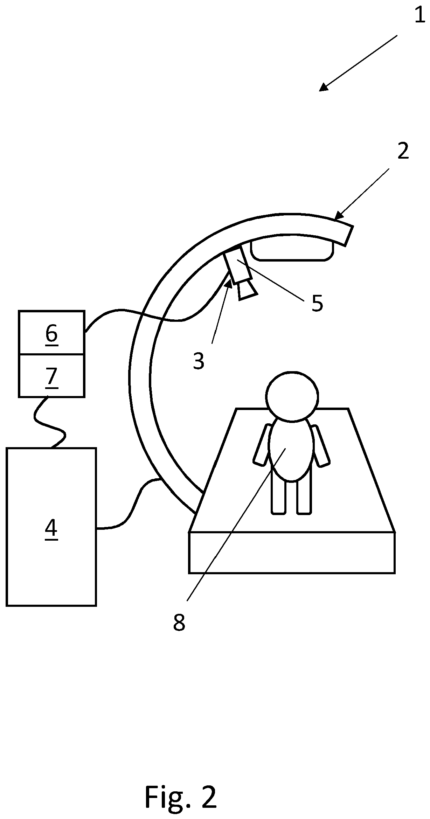

Fig. 2 shows a schematic view of another embodiment of a medical imaging system 1. In this embodiment, the output unit 7 of the patient pose determination system 3 sends the pose of the part of interest to the control unit 4 of the medical imaging device 2. The medical imaging device 2 may then compare the determined pose against an expected pose of the part of interest. If it is determined that there is a mismatch between the determined pose and the expected pose, said mismatch may be indicated and/or a start of a medical scan may be prevented. If the determined pose matches the expected pose, the medical imaging device 2 acquires a medical image and the control unit 4 annotates said medical image with the pose of the part of interest.

Since the determination of the pose and the annotation are automatically performed, an interruption of a workflow of medical staff due to adding manual annotations is avoided. Further, missing annotations or wrong annotations are avoided. Also, lead markers, which are, e.g., critical regarding hospital hygiene, are no longer needed. Further, anatomies that are covered by clothes may be correctly analyzed. Moreover, compared to optical images, the depth images contain little to no patient specific information, making it easier to fulfil data privacy requirements.

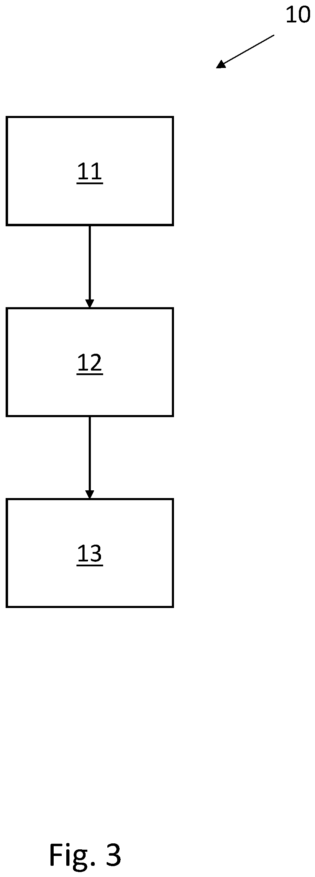

Fig. 3 shows a flowchart of an embodiment of a method 10 of determining a patient pose. The method 10 comprises a step of acquiring 11, by the depth camera 4, a depth image of a part of interest of the patient 8 or another part of the patient 8 pertaining to the part of interest. The method 10 further comprises a step of determining 12, by the processing unit 6, a pose of the part of interest, using the acquired depth image. Finally, the method comprises the step of outputting 13, by the output unit 7, a pose of the part of interest. This method may be, in particular, performed by the patient pose determination system 3 of Fig. 1.

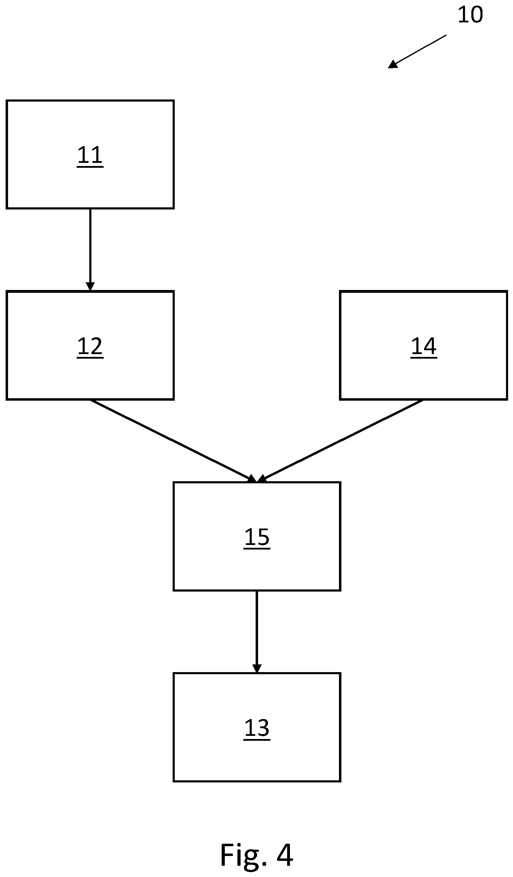

Fig. 4 shows a flowchart of another embodiment of a method 10 of determining a patient pose. In this embodiment, there is a further step of acquiring 14 a medical image of the part of interest by the medical imaging device 2. The determined 12 pose of the part of interest is then used to annotate 15 the acquired 14 medical image. Finally, the step of outputting 13 the pose of the part of interest comprises outputting the pose annotated medical image.

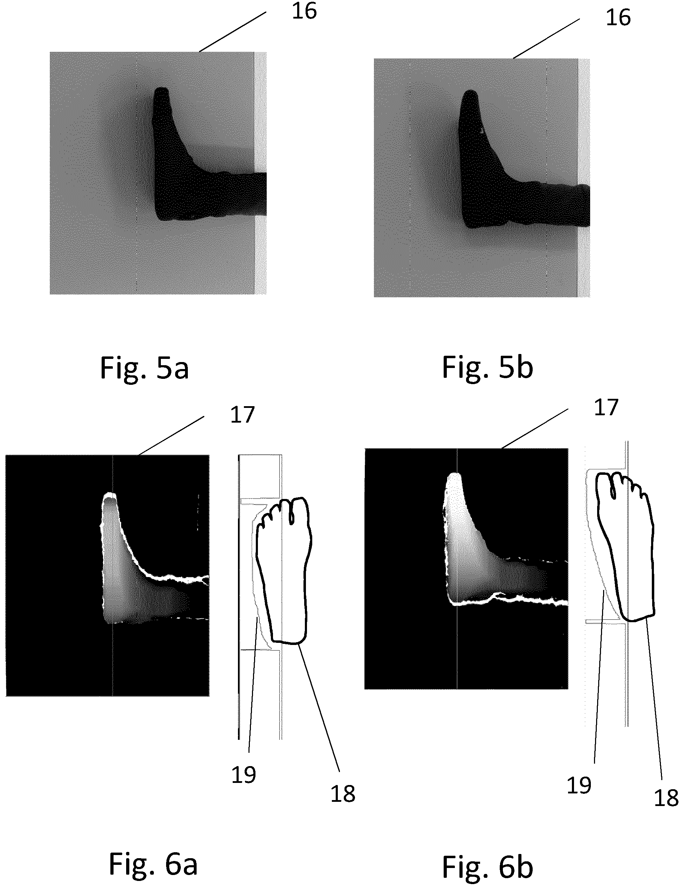

As an example, Fig. 5a shows an optical image 16 of a left foot, covered by a sock. Next to it, Fig. 5b shows an optical image 16 of a right foot, also covered by a sock. Since the socks are black, it is impossible to actually determine the laterality of the feet from the optical images 16. Hence, an automatic patient pose determination system relying on optical images 16 would fail in this case.

However, the depth images 17 of the left foot and the right foot shown in Figs. 6a and 6b, respectively, actually provide information on the laterality of the feet. What can be seen from these depth images 17, e.g., the different heights and curvatures, can as well be analyzed with an artificial intelligence, such as a trained neural network. Hence, contrary to the optical images 16, given the depth images 17, the pose of the part of interest can be determined.

In particular, the determination of the pose may comprise utilization of the artificial intelligence. More particularly, the artificial intelligence may use features and combinations of features in the depth image to determine the pose of the part of interest that are otherwise not easily quantifiable. Further, once the artificial intelligence has been set up, it can be easily adapted for different parts of interest, e.g., by training the artificial intelligence with training data pertaining to said parts of interest. The artificial intelligence may be a trained neural network, in particular a trained convolutional neural network, more particularly a trained fully convolutional neural network. The trained neural networks may have been trained on the basis of depth images of a plurality of parts of interest or other parts in a plurality of known poses. As an example, the depth images may have been labeled with the respective poses.

Next to the depth images 17, models 18 of the left foot and the right foot, respectively, are shown. Also shown are height profiles 19 extracted from the depth images 17. It can be clearly seen that the height profiles 19 match the corresponding models 18, hence, the determination of the laterality could also be performed using said height profiles 19 together with the models 18. In particular, the determination of the pose may comprise analysis of at least one height profile 19 obtained from the depth image 17. Said height profile 19 may be taken along a straight line in the depth image 17, but also along a curved path. Alternatively, or additionally, the determination of the pose may comprise utilization of the model 18 of the part of interest or the other part. Said model may be, e.g., a three-dimensional description of the part of interest or the other part. Depending on the part of interest, anatomy-specific characteristics may be extracted from the depth image 17. These characteristics are then compared to the corresponding characteristics in the model 18 of the part of interest or the other part, and based on said comparison, the pose of the part of interest is determined.

As yet another alternative, the determination of the pose comprises detection of landmarks within the depth image. Said landmarks may be anatomy-specific features that can be easily distinguished from one another. Examples for said landmarks are the nose and the ears for the head, the shoulder, elbow, and fingers for the arm and the knee and the ankle for the leg.

Not shown in the Figures is a computer program product that comprises instructions which, when the program is executed by a computer, cause the computer to carry out the following steps: receiving, from the depth camera 5, the depth image 17 of the part of interest of the patient 8 or another part of the patient 8 pertaining to the part of interest; determining (12) the pose of the part of interest using the depth image 17; and outputting (13) the pose of the part of interest.

Also not shown in the Figures is a computer-readable medium that has stored thereon the computer program product described above.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: medical imaging system
- 2: medical imaging device
- 3: patient pose determination system
- 4: control unit
- 5: depth camera
- 6: processing unit
- 7: output unit
- 8: patient
- 9: screen
- 10: method
- 11: acquiring depth image
- 12: determining
- 13: outputting
- 14: acquiring medical image(s)
- 15: annotating
- 16: optical image
- 17: depth image
- 18: model
- 19: height profile

## Claims

1. A patient pose determination system (3), comprising
a depth camera (5), configured to acquire a depth image (17) of a part of interest of a patient (8) or another part of the patient (8) pertaining to the part of interest;
a processing unit (6), configured to determine a pose of the part of interest using the depth image (17); and
an output unit (7), configured to output the pose of the part of interest.

2. The system (3) according to claim 1, wherein the pose of the part of interest comprises an orientation of the part of interest, a laterality of the part of interest, and/or a view position of the part of interest.

3. The system (3) according to claim 1 or 2, wherein the determination of the pose comprises utilization of an artificial intelligence, in particular of a trained neural network.

4. The system (3) according to claim 3, wherein the trained neural network has been trained on the basis of depth images (17) of a plurality of parts of interest or other parts in a plurality of known poses.

5. The system (3) according to any one of claims 1 to 4, wherein the determination of the pose comprises utilization of a model (18) of the part of interest or the other part.

6. The system (3) according to any one of claims 1 to 5, wherein the determination of the pose comprises detection of landmarks within the depth image (17).

7. The system (3) according to any one of claims 1 to 6, wherein the determination of the pose comprises analysis of at least one height profile (19) obtained from the depth image (17).

8. The system (3) according to any one of claims 1 to 7, wherein outputting the pose of the part of interest comprises displaying information on the pose on a display.

9. The system (3) according to any one of claims 1 to 8, further comprising an optical camera, in particular a color camera, a black-and-white camera and/or an infrared camera, configured to acquire an optical image (16) of the part of interest or the other part of the patient (8),
wherein the processing unit (6) is configured to determine the pose of the part of interest also using the optical image (16).

10. A medical imaging system (1), comprising:
a patient pose determination system (3) according to any one of claims 1 to 9; and
a medical imaging device (2), configured to acquire a medical image of the part of interest,
wherein the processing unit (6) is further configured to annotate the medical image with the pose, and
the output unit (7) is configured to output the pose annotated medical image.

11. The system (1) according to claim 10, wherein the medical imaging device (2) is an X-ray imaging device, a computed tomography, CT, imaging device and/or a magnetic resonance imaging, MRI, device.

12. A method (10) of determining a patient pose, comprising:
acquiring (11), by a depth camera (5), a depth image (17) of a part of interest of a patient (8) or another part of the patient (8) pertaining to the part of interest;
determining (12), by a processing unit (6), a pose of the part of interest using the depth image (17); and
outputting (13), by an output unit (7), the pose of the part of interest.

13. The method (10) according to claim 12, further comprising:
acquiring (14), by a medical imaging device (2), a medical image of the part of interest; and
annotating (15), by the processing unit (6), the medical image with the pose,
wherein outputting (13) the pose of the part of interest comprises outputting the pose annotated medical image.

14. A computer program product comprising instructions which, when the program is executed by a computer, cause the computer to carry out the steps of:
receiving, from a depth camera (5), a depth image (17) of a part of interest of a patient (8) or another part of the patient (8) pertaining to the part of interest;
determining (12) a pose of the part of interest using the depth image (17); and
outputting (13) the pose of the part of interest.

15. A computer-readable medium having stored thereon the computer program product of claim 14.
